# EUROPEAN PATENT APPLICATION

(11) **EP 1 942 337 A1**
(43) Date of publication of application: **09.07.2008**
(21) Application number: 06822256.1
(22) Date of filing: 25.10.2006
(51) Int. Cl.: G01N 27/327, G01N 27/26, G01N 27/416

(54) **SENSOR CHIP AND SENSOR SYSTEM**

(30) Priority: 27.10.2005 JP 2005312326
(71) Applicant: Sumitomo Electric Industries, Ltd., Osaka-shi, Osaka 541-0041 (JP); National Institute of Advanced Industrial Science and Technology, Tokyo 100-8921 (JP)
(72) Inventor: NAKAMURA, Hideaki, Tsukuba-shi Ibaraki 305-8562 (JP); GOTOH, Masao, Tsukuba-shi Ibaraki 305-8562 (JP); ISHIKAWA, Tomoko, Tsukuba-shi Ibaraki 305-8562 (JP); KARUBE, Isao, Tsukuba-shi Ibaraki 305-8562 (JP); HOSOYA, Toshifumi, Osaka-shi Osaka 554-0024 (JP); KAIMORI, Shingo, Osaka-shi Osaka 554-0024 (JP); ICHINO, Moriyasu, Osaka-shi Osaka 554-0024 (JP)
(74) Representative: Cross, Rupert Edward Blount
(86) International application number: PCT/JP2006/321279
(87) International publication number: WO 2007/049646

(57) **Abstract**

It is intended to provide a sensor chip that enables detection of a deterioration state of a reagent applied on a reaction portion when measuring a concentration of a measurement object substance and a sensor system that enables a correct measurement by using the sensor chip for detecting the reagent deterioration state. A sensor chip including two substrates opposed to each other, a spacer layer sandwiched between the substrates, a multiple reaction portions provided in the spacer layer, and detection electrode units disposed on surfaces of the substrates facing to the spacer layer and exposed to the reaction portions, wherein an identical reagent A is applied on two or more of the reaction portions, and another reagent B that reacts with the reagent A is applied on at least one of the two reaction portions and a sensor system including the sensor chip and a measurement unit for comparing current values from the two or more reaction portions on which the identical reagent A is applied.

## Description

### Technical Field

This invention relates to a sensor chip for determining a quantity of a specific component in a sample and, particularly, to a biosensor chip for determining a quantity of a specific component contained in a biological sample. Also, this invention relates to a sensor system using the sensor chip.

### Background Art

A biosensor chip is used for determining a quantity of a substance which is a measurement object by introducing a biological sample such as a blood and a urine into a reaction portion of the chip, causing a biochemical reaction such as an enzyme reaction and an antigen-antibody reaction between a specific component (measurement object substance) contained in the sample and a reagent applied on the reaction portion, and outputting a current value generated by the reaction to outside the chip. The biosensor chip makes use of an excellent molecular discrimination function of living body and attracts attention as a sensor chip that enables a rapid and convenient measurement of a trace quantity of a chemical substance.

In the measurement using the biosensor chip, a concentration of the measurement object substance contained in the sample is determined based on the size of the current value outputted by the reaction portion. However, in the case where the reagent on the reaction portion is deteriorated, the size of the outputted current value is reduced even when the concentration of measurement object substance is not changed, and the measurement becomes difficult when the deterioration is severe. Therefore, in the case where the deterioration of the reagent of the reaction portion is suspected, it is necessary to confirm presence/absence of the deterioration in the measurement.

Recently, importance of regular health management has been pointed out in view of an increase in population of elderly person, and the like, and a demand for an inexpensive and disposable biosensor chip has been increased as an easy measurement means in individual household. However, with such disposable biosensor chip, a problem such as difficulty in correct measurement due to the deterioration of reagent of the reaction portion which occurs depending on storage or the like by the user.

Also, from the view points of reduction in production cost and improvement in usability, it is preferable to pack multiple disposable biosensor chips in one package. However, in such case, since many of the biosensor chips will be exposed to the ambient air for a long time until they are used after the package is opened, the deterioration of reagent tends to occur.

Due to the lack of means to be used by the users for confirming activity of the reagent in the conventional biosensor chips, there is a possibility that a measurement is conducted when the reagent is deteriorated to fail to measure a concentration of a measurement object substance, resulting in erroneous recognition of a correct measurement result. There is a risk that the error in judgment of the measurement result makes it impossible to conduct appropriate self care.

### Disclosure of the Invention

### Problems to be solved by the Invention

An object of this invention is to provide a sensor chip that enables easy detection of a deterioration state of a reagent applied on a reaction portion in the case of using the sensor chip for determining a quantity of a measurement object substance. Another object of this invention is to provide a sensor system that enables a correct measurement by using the sensor chip for detecting the reagent deterioration state.

### Means for solving the Problems

The above objects are attained by a sensor chip (claim 1) including:
two substrates opposed to each other,
a spacer layer sandwiched between the substrates,
a multiple reaction portions provided in the spacer layer, and
detection electrode units disposed on surfaces of the substrates facing to the spacer layer and exposed to the reaction portions, wherein
an identical reagent (hereinafter referred to as reagent A) is applied on two or more of the reaction portions, and
another reagent (hereinafter referred to as reagent B) that reacts with the reagent is applied on at least one of the two reaction portions.

As a result of study, the inventor of this invention find that, by using a sensor chip wherein an identical reagent is applied on two or more of reaction portions and another reagent that reacts with the aforementioned reagent is applied on at least one of the two reaction portions, it is possible to easily detect a degree of deterioration of the reagent by comparing current values outputted from the reaction portions.

The sensor chip of this invention includes two substrates opposed, i.e. facing, to each other, and a spacer layer sandwiched between the substrates. On one side of each of the substrates facing to the spacer layer, i.e. on each of surfaces facing to each other, a detection electrode unit is formed. Each of the detection electrode units has at least an active electrode and a counter electrode and may have another electrode such as a reference electrode and other means as required. The active electrode and the counter electrode in each of the detection electrode units are ordinarily disposed in parallel to each other on the surface of the substrate. The detection electrode units are respectively exposed to the reaction portions to output current values generated due to reactions in the reaction portions.

The two substrates are electrically insulated films, and materials therefor include ceramics, glass, a paper, a biodegradable material (e.g. polylactic acid, starch, etc.), a plastic material such as polyethylene telephthalate, and the like, wherein the insulating resin such as polyethylene telephthalate may preferably be used.

The spacer layer sandwiched between the two substrates is formed of one or more layers, and examples thereof include a resist layer having a function of improving a property for insulation between the electrodes and physically protecting the electrodes and a layer formed of multiple layers such as a tackiness agent layer, an adhesive agent layer, and the like having a function of attaching layers to each other. Also, one layer may be used as the resist layer and the adhesive agent layer, and each of the resist layer, the tackiness agent layer, and the adhesive agent layer may be formed of multiple layers.

The resist layer serves as a spacer after formation of the sensor chip, and due to strong adhesion to the substrates, has a function of preventing boundary surfaces between the substrates and the spacer layer from peeling in the case where a liquid reagent which is applied on the reaction portions permeates into the boundary surfaces upon the application.

Examples of a material for the resist layer include a urethane resin, an epoxy resin, a modified polyimide resin, an acryl resin, and the like. Examples for a tackiness agent for forming the tackiness agent layer include a rubber-based tackiness agent, an acryl-based tackiness agent, and a silicone-based tackiness agent. Examples of an adhesive agent for forming the adhesive agent layer include epoxy-based, vinyl acetate-based, silicone-based adhesive agents, and the like, and a heat curable resin such as an epoxy resin, a UV curable resin, and the like may be used.

Although the adhesive agent layer and the tackiness agent layer also serve as spacers after formation of the sensor chip, since the adhesive agent and the tackiness agent in general have a low young's modulus and are easily deformed, it is undesirable to increase a thickness thereof by a large scale from the view point of restriction of a volume of the reaction portions. The thickness may ordinarily preferably be as small as possible in the range of thickness enabling the function of adhering by both sides.

In the sensor chip of this invention, two or more reaction portions are provided in the spacer layer. More preferably, the sensor chip has at least two reaction portions wherein: the detection electrode unit is provided on the surface of each of the two substrates facing to the spacer layer; the detection electrode unit formed on the surface of one of the substrates (substrate 1) is exposed, while the detection electrode unit on the other substrate (substrate 2) being not exposed in one of the reaction portions; and the detection electrode unit of the substrate 2 is exposed, while the detection electrode unit of the substrate 1 being not exposed in the other reaction portion. By the above-described facing structure of the detection electrode unit of the substrate 1 and the detection electrode unit of the substrate 2, it is possible to keep the size of the sensor chip of this invention compact since multiple electrode units are not aligned on one surface.

The reaction portion is a hollow portion provided in the spacer layer, and a chemical reaction (biochemical reaction in the case of a biosensor chip) between a sample introduced into the reaction portion and a reagent applied on the reaction portion is caused for detection and quantity determination in the reaction portion. Examples of the reagent include a catalyst, an enzyme, and the like for accelerating the chemical reaction, and the reagent is applied and immobilized inside the reaction portion.

Examples of the catalyst and the enzyme to be used as the reagent include glucose oxidase (GOD), glucose dehydrogenase (GDH), a glucose oxidase-electron receptor (mediator) mixture, a glucose oxidase-albumin mixture, a glucose oxidase-electron receptor-albumin mixture, and the like in the case of a glucose biosensor for measuring a glucose amount in blood; a fructose dehydrogenase (FDH) in the case of a fructose sensor; lactic acid oxidase in the case of a lactic acid sensor; cholesterol oxidase in the case of a cholesterol sensor; alcohol oxidase in the case of an alcohol sensor; amino acid oxidase in the case of an amino acid sensor; and the like. In order to perform a smooth reaction, a surfactant or the like may be applied in some cases.

It is necessary that the reaction portion has at least one sample inlet at one end thereof. Also, an opening may preferably be formed on the end opposite to the sample inlet. By forming such straw-like structure of the reaction portion, it is possible to facilitate charging of the sample to the reaction portion by using a capillary phenomenon.

The sensor chip of this invention is characterized by including, among the multiple reaction portions, the reaction portion on which a reagent A is applied and the reaction portion on which the reagent A and another reagent B that reacts with the reagent A are applied. Hereinafter, the reaction portions on which only the reagent A is applied will be referred to as a measurement reaction portion, the reaction portion on which the reagent A and the reagent B are applied will be referred to as a reference reaction portion, and the reagent B will be referred to as a reference reagent.

It is possible to form the reaction portions by applying the identical reagent A on each of two reaction portions, and applying the reference reagent (reagent B) on one of the two reaction portions at a portion different from a portion on which the reagent A is applied in a step of reagent application in sensor chip production.

When the sample is introduced into the measurement reaction portion and the reference reaction portion, a measurement object substance in the sample is brought into reaction by the reagent A in the measurement reaction portion so that a current value is outputted to the detection electrode unit. This current value will be referred to as Ia. In the reference reaction portion, the measurement object substance in the sample is brought into reaction by the reagent A, and the reference reagent reacts with the reagent A, so that a current value is outputted to the detection electrode unit. This current value will be referred to as Ib. The current value Ib is a sum of the current value (equal to Ia) caused by the reaction between the measurement object substance in the sample and the reagent A and the current value due to the reaction between the reference reagent and the reagent A, a difference Ix = (Ib-Ia) between the outputted current values is a current value due to the reaction between the reference reagent and the reagent A.

When the reagent A is deteriorated, the reaction between the reference reagent and the reagent A becomes inactive, so that Ix is diminished with the deterioration of the reagent A. Therefore, it is possible to judge a deterioration state of the reagent A by comparing a current value Io due to the reaction between the reference reagent and the reagent A with Ix when the reagent A is not deteriorated. It is possible to obtain the current value Io by preliminary measuring Ia and Ib of a sample containing a predetermined concentration of the measurement object substance immediately after designing or production of the sensor chip, i.e. when the reagent A is not deteriorated at all.

The reference reagent (reagent B) to be used is not particularly limited insofar as the reagent reacts with the reagent A with high activity and causes a current value responsive to the reaction to be outputted, and it is more preferable to use a substance which is identical to the measurement object substance (claim 2). By using an identical substance for the reference reagent and the measurement object substance, it is possible to accurately extract the change in output based on the change in activity of the reagent A.

The sensor chip of this invention may have a reaction portion other than the measurement reaction portion and the reference reaction portion. By applying on the another reaction portion a reagent other than the reagent A applied on the measurement reaction portion and the reference reaction portion, it is possible to simultaneously measure different components in the sample.

The sensor chip of this invention is suitably used as a biosensor chip. Claim 3 corresponds to this preferred mode. The sensor chip is usable as, for example, a blood sugar level sensor, a urinary sugar level sensor, and the like for measuring a glucose amount (blood sugar level) in blood and suitably used as a fructose sensor, checkups for hepatic function, checkups for neutral fat and a cholesterol amount, and the like. Particularly, the sensor chip is suitably used as a disposable biochip which is subject to the problem of reagent deterioration.

In the case of determining a quantity of a measurement object substance in a sample by the sensor chip of this invention, the sensor chip is mounted on a sensor device, and the sample inlet of the sensor chip is brought into contact with the sample to introduce the sample into the reaction portion for reaction. The sensor device includes a terminal for inputting current values from the sensor chip, a unit for calculating a measurement result by processing the current values, a unit for displaying the measurement result, and the like, and a sensor system for determining a quantity of a measurement object substance in a sample is formed by the sensor chip and the sensor device.

In addition to the above sensor chip, this invention provides a sensor system which is formed of the above sensor chip and a sensor device. That is, there is provided a sensor system (claim 4) including:
a sensor chip including:
   two substrates opposed to each other,
   a spacer layer sandwiched between the substrates,
   a multiple reaction portions provided in the spacer layer, and
   detection electrode units disposed on surfaces of the substrates facing to the spacer layer and exposed to the reaction portions, wherein
   an identical reagent (hereinafter referred to as reagent A) is applied on two or more of the reaction portions, and
   another reagent (hereinafter referred to as reagent B) that reacts with the reagent A is applied on at least one of the two reaction portions, and
a measurement unit for comparing current values from the two or more reaction portions on which the identical reagent A is applied.

As described above, the sensor system of this invention is characterized by using the sensor chip of this invention and including the measurement unit for comparing the current values outputted from the measurement reaction portion an the reference reaction portion, i.e. Ia and Ib. The measurement unit that conducts the comparison obtains Ix that is the difference between Ia and Ib.

When the current value Io preliminary measured as described above is stored in a storage of the sensor device, it is possible to obtain Ix/Io, so that a deterioration state of the reagent A is recognized depending on a reduction in Ix/Io.

In the case where an activity reduction in the reagent A is confirmed, use of the sensor chip is prohibited, for example, so that the sensor chip is replaced with another sensor chip to conduct a measurement. In this case, it is preferable that recommendation for replacement is outputted on the display unit of the sensor device.

It is also possible to employ a method of determining a quantity of a measurement object substance by: conducting a measurement by using the sensor chip with which the reduction in activity of the reagent A has been confirmed; and correcting the current value Ia based on the value of Ix/Io. With such method, it is possible to prevent a loss due to disposal and replacement of the sensor chips and an increase in cost. Also, it is possible to employ a method of determining a quantity of a measurement object substance by: setting a predetermined threshold value Id; judging that deterioration of the reagent A is prominent when the current value Ix is below Id and replacing the sensor chip; and correcting the current value Ia based on the value of Ix/Io when the current value Ix is equal to or above Id.

### Effect of the Invention

By using the sensor chip and the sensor system of this invention, it is possible to easily judge a deterioration state of the reagent A applied on the reaction portions when conducting a measurement. Therefore, in the case where activity of the reagent A is reduced due to storage by a user, it is possible to conduct correct quantity determination by appropriately recognizing a degree of the reduction and employing a method of replacing the sensor chip or performing correction on outputs based on the degree of reduction, thereby avoiding inappropriate judgment due to the use of the deteriorated sensor chip.

Also, since the sensor chip of this invention does not require an increase in chip size and does not have component members having complicated shape, production thereof is easy. Therefore, it is possible to use the sensor chip as a disposable biosensor chip or the like to be suitably used for regular health management in individual household.

### Brief Description of Drawings

[Fig. 1]

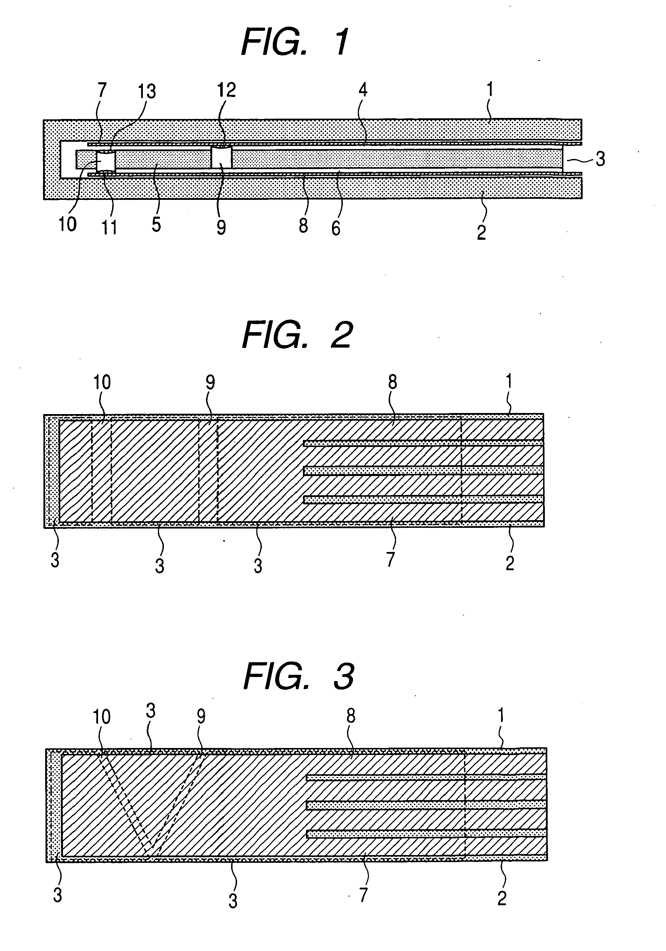

   Fig. 1 is a side view showing one example of sensor chip of this invention.
[Fig. 2]
   Fig. 2 is a plan view showing one example of sensor chip of this invention.
[Fig. 3]
   Fig. 3 is a plan view showing another example of sensor chip of this invention.
[Fig. 4]

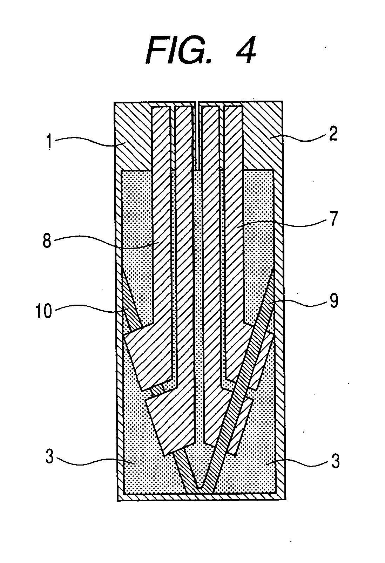

   Fig. 4 is a plan view showing another example of sensor chip of this invention.

### Description of Reference Numerals and Signs

1, 2: substrates
3: spacer layer
4, 6: resist layer
5: adhesive agent layer
7, 8: detection electrode units
9: measurement reaction portion
10: reference reaction portion
11, 12: reagent A
13: reference reagent

### Best Mode for Carrying out the Invention

Hereinafter, the best mode for carrying out this invention will be described by using the drawings. This invention is not limited to the mode and can be altered into another mode insofar as the effect of this invention is not diminished.

One example of sensor chip of this invention is shown in Figs. 1 and 2, wherein shown in Fig. 1 is a side view, and shown in Fig. 2 is a plan view.

As shown in Fig. 1, a spacer layer 3 sandwiched between a substrate 1 and a substrate 2 is formed of a resist layer 4, a tackiness agent layer 5, and a resist layer 6. A detection electrode unit 7 is formed on a surface of the substrate 1 facing to the spacer layer 3, and a detection electrode unit 8 is formed on a surface of the substrate 2 facing to the spacer layer 3.

In the spacer layer 3, a measurement reaction portion 9 and a reference reaction portion 10 are formed. That is, shown in Figs. 1 and 2 is a biosensor chip having two reaction portions wherein, one of them is the measurement reaction portion and the other one is the reference reaction portion.

The measurement reaction portion 9 is formed in the resist layer 4 and the tackiness agent layer 5, and only the detection electrode unit 7 is exposed to the measurement reaction portion 9. The reference reaction portion 10 is formed in the tackiness agent layer 5 and the resist layer 6, and only the detection electrode unit 8 is exposed to the reference reaction portion 10.

As shown in Fig. 2, the measurement reaction portion 9 and the reference reaction portion 10 are parallel to each other, and both ends of each of the measurement reaction portion 9 and the reference reaction portion 10 are opened at both sides of the sensor chip. Therefore, each of the measurement reaction portion 9 and the reference reaction portion 10 has a straw-like shape, and the ends of each of the reaction portions is used as a sample inlet from which a sample is introduced.

An identical reagent A 11 and 12 is immobilized on the measurement reaction portion 9 and the reference reaction portion 10. A reference reagent 13 (reagent B) is immobilized on the reference reaction portion 10 at a portion different from the portion on which the reagent A 11 is immobilized and separately from the reagent A 11. In this example, the reference reagent 13 is immobilized on a surface opposed to a surface on which the reagent A 11 is immobilized inside the reaction portion.

Hereinafter, a production method for the sensor chip of Figs. 1 and 2 will be described.

After formation of the sensor chip, the detection electrode units 7 and 8 are formed on a substrate sheet to be used as the substrate 1 and the substrate 2. It is possible to form the detection electrode units 7 and 8 by screen-printing a carbon ink, and the detection electrode unit 7 is formed on one of two parts defined by a folding line at which the substrate sheet can be substantially divided into half, and the detection electrode unit 8 is formed on the other part.

After the formation of the detection electrode units 7 and 8, the resist layers 4 and 6 are formed on the detection electrode units 7 and 8. The resist layers 4 and 6 are formed in such a manner as to have grooves each serving as a part of the measurement reaction portion 9 and a part of the reference reaction portion 10 after the sensor chip formation. The grooves are parallel to each other and perpendicular to sides of the substrate sheet, and the detection electrode units 7 and 8 are respectively exposed to the grooves. Such resist layers 4 an 6 are formed by employing a method of curing a resin forming the resist layers 4 and 6 by screen printing in such a manner as to form the grooves.

After forming the resist layers 4 and 6, tackiness agent layers are formed. The tackiness agent layers are formed in such a manner as to form grooves at positions overlapping with the above-described two grooves and to form groves at positions symmetrical to the grooves about the folding line. The tackiness agent layers are formed by employing a method of screen-printing a resin forming the tackiness agent layer in the same manner as in the above-described groove formation method.

After the grooves are formed as described above, the reagent A and the reference reagent are applied on bottoms of the grooves. After the application of the reagent A and the reference reagent, the substrate sheet is folded at the folding line to attach the tackiness agent layers to each other. As a result, the attached tackiness agent layers form the tackiness agent layer 5, and each of the grooves forms the measurement reaction portion 9 and the reference reaction portion 10, thereby obtaining the sensor chip.

Figs. 3 and 4 are plan views showing other examples of sensor chip of this invention.

In Figs. 3 and 4, a measurement reaction portion 9 and a reference reaction portion 10 are disposed in such a fashion that a V-shape is formed by the measurement reaction portion 9 and the reference reaction portion 10. Both ends of each of the measurement reaction portion 9 and the reference reaction portion 10 are opened at both sides of the sensor chip to achieve a straw-like shape, and the openings are connected at one side. As a result, by using the connected opening as a sample inlet, it is possible to simultaneously introduce a very small amount of a sample into both of the measurement reaction portion 9 and the reference reaction portion 10.

In each of the examples of Figs. 3 and 4, the measurement reaction portion 9 is formed in a spacer layer 3, and only the detection electrode unit 7 is exposed to the measurement reaction portion 9. Also, the reference reaction portion 10 is formed in the spacer layer 3, and only the detection electrode unit 8 is exposed to the reference reaction portion 10. An identical reagent A is immobilized on the measurement reaction portion 9 and the reference reaction portion 10, and a reference reagent is immobilized on the reference reaction portion 10 at a portion different from the portion at which the reagent A is immobilized. Since these component portions of the examples are the same as those of the example of Fig. 2 and can be formed in the same manner as in the example of Fig. 2, detailed descriptions thereof are omitted.

Though this invention is described in detail with reference to the specific embodiments in the foregoing, it is apparent for the skilled in the art that it is possible to add various alterations and modifications without departing from the scope and the spirit of this invention. This patent application is based on the Japanese Patent Application (JP-2005-312326) filed on October 27, 2005, and contents thereof is incorporated herein by reference.

## Claims

1. A sensor chip comprising:
two substrates opposed to each other,
a spacer layer sandwiched between the substrates,
a multiple reaction portions provided in the spacer layer, and
detection electrode units disposed on surfaces of the substrates facing to the spacer layer and exposed to the reaction portions, wherein
an identical reagent A is applied on two or more of the reaction portions, and
another reagent B that reacts with the reagent A is applied on at least one of the two reaction portions.

2. The sensor chip according to claim 1, wherein
the reagent B is a measurement object substance.

3. The sensor chip according to claim 1 or 2, which is a biochip.

4. A sensor system comprising:
a sensor chip comprising:
two substrates opposed to each other,
a spacer layer sandwiched between the substrates,
a multiple reaction portions provided in the spacer layer, and
detection electrode units disposed on surfaces of the substrates facing to the spacer layer and exposed to the reaction portions, wherein
an identical reagent A is applied on two or more of the reaction portions, and
another reagent B that reacts with the reagent A is applied on at least one of the two reaction portions, and
a measurement unit for comparing current values from the two or more reaction portions on which the identical reagent A is applied.
